# EUROPEAN PATENT SPECIFICATION

(11) **EP 3 983 034 B1**
(45) Date of publication and mention of the grant of the patent: **01.04.2026**
(21) Application number: 20733916.9
(22) Date of filing: 13.06.2020
(51) Int. Cl.: A61M 5/28, A61M 5/315

(54) **IMPROVED DRUG DELIVERY DEVICE HAVING A PLUNGER WITH ADJUSTABLE LENGTH**
VERBESSERTE WIRKSTOFFFREISETZUNGSVORRICHTUNG MIT EINEM KOLBEN MIT EINSTELLBARER LÄNGE
DISPOSITIF D'ADMINISTRATION AMÉLIORÉ DE MÉDICAMENT AYANT UN PISTON À LONGUEUR RÉGLABLE

(30) Priority: 14.06.2019 GB 201908597
(43) Date of publication of application: 20.04.2022
(73) Proprietor: Owen MumFord Ltd., Woodstock Oxfordshire OX20 1TU (GB)
(72) Inventor: SHABUDIN, Tahir, Woodstock Oxfordshire OX20 1TU (GB)
(74) Representative: Marks & Clerk LLP
(86) International application number: PCT/EP2020/066414
(87) International publication number: WO 2020/249807

(56) References cited:
- EP-A1- 3 337 538
- EP-A1- 3 628 351
- EP-B1- 3 337 538
- WO-A1-2009/108869
- GB-A- 2 546 499
- US-A- 5 591 136

## Description

### Technical field

The invention relates to pre-filled syringes and methods of assembly of pre-filled syringes.

### Background

Broadly, syringes comprise a barrel having a hypodermic needle at one end and a plunger configured to move within the barrel such that an inward stroke of the plunger causes a substance contained within the barrel to be expelled from the needle.

Syringes may further include some form of safety mechanism to protect healthcare workers from the hypodermic needle after the substance has been injected into a patient. Exemplary syringes may include a sheath for covering the needle, or may cause the needle to retract within the barrel of the syringe.

Syringes may be provided to a user pre-filled with a substance (such as a medicament), such that they are ready to use. Pre-filled syringes may therefore be transported to a user with the medicament already contained within the barrel. The fill volume of the pre-filled syringe may vary depending on the dosage of medicament to be dispensed to a user. EP3337538A1 relates to an injection device for injection of an accurate dose of medicament. US5591136A relates to a safety injection device. GB2546499A relates to an apparatus for use with a syringe for providing a safety syringe. WO2009/108869A1 relates to a syringe with an adjustable two piece plunger rod. EP3628351A1 relates to an injection device with fill volume management.

### Summary

According to the invention in an aspect, there is provided a method of assembling a pre-filled syringe according to claim 1. Also disclosed herein are methods of assembling a pre-filled syringe comprising: setting a length of a plunger rod of a plunger rod assembly; and fitting the plunger rod assembly to a barrel of the syringe, the barrel having an opening at a forward end thereof and comprising a bung positioned therein and creating a volume between the bung and the opening, the volume at least partially filled with a substance for delivery from the syringe, wherein setting the length of the plunger rod assembly comprises at least partially inserting the plunger rod into the barrel such that a forward end of the plunger rod contacts the bung, and wherein, after assembly, the length of the plunger rod defines a separation between the bung and the forward end of the plunger rod. The separation may accommodate expansion of the substance within the barrel.

As used herein, the term "fitting" with respect to the plunger assembly being fitted to the barrel encompasses fitting directly and/or indirectly. That is, the plunger rod assembly may be fitted to a handle portion that is in turn fitted to the barrel.

Optionally, setting the length of the plunger rod comprises positioning a first portion of the plunger rod with respect to a second portion of the plunger rod.

Optionally, the first and second portions of the plunger rod are coupled and the first portion is axially moveable with respect to the second portion, and wherein positioning the first portion with respect to the second portion comprises axially moving the first portion with respect to the second portion.

Optionally, the method comprises further insertion of the plunger rod into the barrel, wherein the further insertion results in an axial force acting on the first portion for positioning the first portion with respect to the second portion. It is noted that, because the first portion is axially movable with respect to the second portion, further insertion of the plunger rod into the barrel results in axial compression of the plunger rod. In effect, further insertion of the plunger rod may be considered further insertion of the second portion.

Optionally, the first and second portions is telescopically received in the other of the first and second portions.

Optionally, setting the length of the plunger rod comprises rotating at least one of the first portion and second portion with respect to the other of the first portion and the second portion.

Optionally, setting the length of the plunger rod comprises engaging reciprocal threads of the first portion and the second portion for translating rotational force into axial force and vice-versa, and rotating one of the first portion and second portion with respect to the other of the first portion and the second portion.

Optionally, the first portion is a forward portion and the second portion is a rearward portion, the method further comprising: engaging the first portion with a rotational lock to rotationally fix the first portion relative to the barrel before further insertion of the plunger rod into the barrel, such that the second portion rotates relative to the first portion.

Optionally, the rotational lock comprises a keyed aperture, a cross section of at least part of the plunger rod corresponding to a shape of the keyed aperture.

Optionally, the method further comprises rotationally fixing the second portion with respect to the first portion after fitting the plunger rod assembly to the barrel.

The method further comprises moving the plunger rod rearward after setting the length of the plunger rod for defining the separation between the bung and the forward end of the plunger rod.

Optionally, the plunger rod assembly further comprises a rearward member and at least one leg, the at least one leg slidably engaged, directly or indirectly, with the barrel after fitting of the plunger rod assembly to the barrel, and wherein rotationally fixing the second portion with respect to the first portion comprises engaging the second portion with the rearward member.

Optionally, the plunger rod assembly further comprising a forward member for fitting to the barrel and slidably engaged with the at least one leg, the forward member comprising the rotational lock.

Optionally, engaging the second portion with the rearward member comprises moving the plunger rod rearward.

Optionally, the rearward member comprises at least one aperture for receiving at least one rearwardly extending prong of the second portion, and wherein engaging the second portion with the rearward member comprises inserting the at least one prong into the at least one aperture.

Optionally, the method further comprises preventing insertion of the at least one prong into the at least one aperture during further insertion of the plunger rod into the barrel.

Optionally, preventing insertion of the at least one prong into the at least one aperture comprises obscuring the at least one aperture.

Optionally, obscuring the at least one aperture comprises inserting a stopper into the at least one aperture to prevent the prongs of the rearward portion from being inserted into the at least one aperture.

Optionally, the method further comprises removing the stopper from the at least one aperture after fitting the plunger rod assembly to the barrel to allow receipt of the at least one prong within the at least one aperture.

Also disclosed herein is a method of assembling a pre-filled syringe comprising: rotating a first portion of a plunger rod of a plunger rod assembly with respect to a second portion of the plunger rod, the first portion and the second portion comprising reciprocal threads for translating rotational force into axial force; setting a length of the plunger rod by rotationally fixing the first portion with respect to the second portion; and fitting the plunger rod assembly to a barrel of the syringe, the barrel having an opening at a forward end thereof and comprising a bung positioned therein and creating a volume between the bung and the opening, the volume at least partially filled with a substance for delivery from the syringe, wherein, after assembly, the length of the plunger rod defines a separation between the bung and a forward end of the plunger rod. The separation may accommodate expansion of the substance within the barrel.

Optionally, the plunger rod assembly further comprises a rearward member and at least one leg, the at least one leg slidably engaged, directly or indirectly, with the barrel after fitting of the plunger rod assembly to the barrel, and wherein the first portion is a forward portion rotatable with respect to the second portion before setting the length of the plunger, which is a rearward portion and rotationally fixed with respect to the rearward member.

Optionally, rotationally fixing the first portion comprises engaging the first portion with a rotational lock after setting the length of the plunger rod.

Optionally, the plunger rod assembly further comprises a forward member for fitting to the barrel and slidably engaged with the at least one leg, the forward member comprising the rotational lock.

Optionally, the rotational lock comprises a keyed aperture, a cross section of at least part of the plunger rod corresponding to a shape of the keyed aperture.

Optionally, the forward member is engaged with the first portion of the plunger rod, the method further comprising sliding the forward member along the at least one leg to disengage the forward member from the first portion before rotating the first portion.

Also disclosed herein is a method of assembling a pre-filled syringe comprising: rotating a first portion of a plunger rod of a plunger rod assembly with respect to a second portion of the plunger rod, the first portion and the second portion comprising reciprocal threads for translating rotational force into axial force; setting a length of the plunger rod of by rotationally fixing the first portion with respect to the second portion in a first direction, and allowing rotation of the first portion with respect to the second portion in a second direction; and fitting the plunger rod assembly to a barrel of the syringe, the barrel having an opening at a forward end thereof and comprising a bung positioned therein and creating a volume between the bung and the opening, the volume at least partially filled with a substance for delivery from the syringe, wherein, after assembly, the length of the plunger rod defines a separation between the bung and a forward end of the plunger rod. The separation may accommodate expansion of the substance within the barrel.

Optionally, rotationally fixing the first portion with respect to the second portion in a first direction, and allowing rotation of the first portion with respect to the second portion in a second direction comprises engaging the first portion of the plunger rod with a ratchet.

Optionally, the reciprocal threads are arranged to lengthen the plunger rod when a rotational force in the second direction is applied to the first portion of the plunger rod.

Optionally, the reciprocal threads are arranged to produce a rotational force in the first direction when a forward axial force is applied to the second portion of the plunger rod.

Also disclosed herein is a pre-filled syringe comprising:
a barrel having an opening at a forward end thereof;
a bung positioned within the barrel to create a volume between the bung and the forward opening, the volume at least partially filled with a substance for delivery from the syringe; and
a plunger rod assembly comprising a plunger rod having an adjustable length configured such that a forward end of the plunger rod is in contact with the bung on fitting the plunger rod assembly to the barrel,
and wherein, when the plunger rod assembly is fitted to the barrel, the plunger rod is moveable rearwards with respect to the barrel to define a separation between the bung and the forward end of the plunger.

Optionally, the plunger rod comprises a first portion coupled to a second portion, and wherein the first portion is axially moveable with respect to the second portion to adjust the length of the plunger rod.

Optionally, one of the first and second portions is telescopically received in the other of the first and second portions.

Optionally, at least one of the first portion and second portion is rotatable with respect to the other of the first portion and second portion.

Optionally, the first and second portions comprise reciprocal threads for translating rotational force into axial force and vice-versa.

Optionally, the first portion is a forward portion and the second portion is a rearward portion, and wherein the pre-filled syringe further comprises a rotational lock configured to rotationally fix the first portion relative to the barrel.

Optionally, the rotational lock comprises a keyed aperture and wherein a cross section of at least part of the plunger rod corresponds to a shape of the keyed aperture.

Optionally, the plunger rod assembly further comprises a rearward member configured to engage the second portion to rotationally fix the second portion with respect to the first portion.

Optionally, the rearward member is configured to engage the second portion when the plunger rod is moved rearward with respect to the barrel after the plunger rod assembly is fitted thereto

Optionally, the rearward member comprises at least one aperture configured to receive at least one rearwardly extending prong of the second portion, and wherein receipt of the at least one rearwardly extending prong within the at least one aperture rotationally fixes the second portion with respect to the first portion.

Also disclosed herein is a pre-filled syringe comprising a stopper configured to selectively obscure the at least one aperture to prevent the prongs of the rearward portion from being inserted into the at least one aperture.

Optionally, the plunger rod assembly further comprises at least one leg configured to be slidably engaged, directly or indirectly, with the barrel after fitting of the plunger rod assembly to the barrel.

Optionally, the plunger rod assembly further comprises a forward member configured to be fitted to the barrel and comprising the rotational lock, wherein the forward member is configured to be slidably engaged with the at least one leg.

Also disclosed herein is a plunger rod assembly for use with a pre-filled syringe comprising a barrel having an opening at a forward end thereof and a bung positioned within the barrel to create a volume between the bung and the forward opening, the volume at least partially filled with a substance for delivery from the syringe, the plunger rod assembly comprising: a plunger rod having an adjustable length configured such that a forward end of the plunger rod is in contact with the bung on fitting the plunger rod assembly to the barrel, wherein, when the plunger rod assembly is fitted to the barrel, the plunger rod is moveable rearwards with respect to the barrel to define a separation between the bung and the forward end of the plunger.

Also disclosed herein is a pre-filled syringe comprising: a barrel having an opening at a forward end thereof; a bung positioned within the barrel to create a volume between the bung and the forward opening, the volume at least partially filled with a substance for delivery from the syringe; and a plunger rod assembly comprising a plunger rod having a first portion and a second portion, the first and second portions comprising reciprocal threads configured to translate relative rotation between the first and second portions into relative axial displacement between the first and second portions; and a rotational lock configured to rotationally fix the first portion with respect to the second portion wherein the plunger rod assembly is configured to be fitted to the barrel and wherein the plunger rod length is adjustable such that, when the plunger rod assembly is fitted to the barrel, a forward end of the plunger rod is separated from the bung.

Optionally, the plunger rod assembly further comprises a rearward member and at least one leg, the at least one leg slidably engaged, directly or indirectly, with the barrel after fitting of the plunger rod assembly to the barrel, and wherein the first portion is a forward portion rotatable with respect to the second portion, which is a rearward portion and rotationally fixed with respect to the rearward member.

Optionally, the plunger rod assembly further comprises a forward member for fitting to the barrel and slidably engaged with the at least one leg, the forward member comprising the rotational lock.

Optionally, the rotational lock comprises a keyed aperture, a cross section of at least part of the plunger rod corresponding to a shape of the keyed aperture.

Optionally, the forward member is slidable rearwards along the at least one leg to release the rotational lock.

Optionally, when engaged the rotational lock fixes the first portion with respect to the second portion in a first direction, and allows rotation of the first portion with respect to the second portion in a second direction.

Optionally, the rotational lock comprises a ratchet.

Optionally, the reciprocal threads are arranged to lengthen the plunger rod when a rotational force in the second direction is applied to the first portion of the plunger rod.

Optionally, the reciprocal threads are arranged to produce a rotational force in the first direction when a forward axial force is applied to the second portion of the plunger rod.

Also disclosed herein is a plunger rod assembly for use with a pre-filled syringe comprising a barrel having an opening at a forward end thereof and a bung positioned within the barrel to create a volume between the bung and the forward opening, the volume at least partially filled with a substance for delivery from the syringe, the plunger rod assembly comprising: a plunger rod having a first portion and a second portion, the first and second portions comprising reciprocal threads configured to translate relative rotation between the first and second portions into relative axial displacement between the first and second portions; and a rotational lock configured to rotationally fix the first portion with respect to the second portion wherein the plunger rod assembly is configured to be fitted to the barrel and wherein the plunger rod length is adjustable such that, when the plunger rod assembly is fitted to the barrel, a forward end of the plunger rod is separated from the bung.

According to the invention in an aspect, there is provided a kit of parts comprising the plunger rod assembly according to any disclosed herein (in particular those disclosed above) and a pre-filled syringe.

According to the invention in an aspect, there is provided a kit of parts comprising the plunger rod assembly according to any disclosed herein (in particular those disclosed above) and a forward assembly.

### Brief description of the drawings

Figure 1 is an exploded view of an exemplary pre-filled syringe;
Figure 2 is a partial section view of an exemplary plunger rod assembly;
Figures 3a-3i show an exemplary pre-filled syringe at different stages of assembly;
Figure 4 shows an exemplary plunger rod assembly;
Figure 5 shows a partial section view of the exemplary plunger rod assembly of Figure 4; and
Figures 6a-g show an exemplary pre-filled syringe at different stages of assembly.

### Detailed description

Generally disclosed herein are pre-filled syringes comprising a barrel having an opening at a forward end thereof and a bung positioned therein. A volume may be created between the bung and the opening, the volume at least partially filled with a substance to be delivered from the syringe. The pre-filled syringe may further comprise a plunger rod moveable within the barrel to drive the bung forwards to deliver the substance through the opening of the barrel. For the remainder of this document, the substance is referred to as a medicament for clarity. This need not be seen as limiting on the scope of the invention as claimed.

Typically, assembled pre-filled syringes may be provided to a user in a ready to use state, in which a forward end of the plunger rod is held in contact with the bung, and the bung, in turn, is held in contact with the substance contained within the barrel. The plunger rod and the bung may be held in position such that rearward movement away from the medicament is not possible in an assembled (or pre-use) configuration.

The inventors have realised that transporting such pre-filled syringes in low-pressure environments (such as at high altitude) may cause leakage of the medicament, since if the plunger rod and the bung are fixed in position, there is no volume into which the medicament can expand.

Exemplary pre-filled syringes may therefore comprise a separation between the bung and a forward end of a plunger rod of a plunger rod assembly when in an assembled condition. In exemplary pre-filled syringes, the separation between the bung and the forward end of the plunger rod accommodates expansion of a medicament in a barrel of the pre-filled syringe, which prevents leaks.

Throughout the specification, the term "forward" refers to the end of the safety syringe from which the medicament is delivered. In other words, the forward end of the safety syringe is the end proximal to an injection site during use. The terms "rear" or "rearward" refer to the plunger end of the safety syringe or component thereof. In other words, the term "rearward" means distant or remote from the injection site during use. Other relative terms such as axial, longitudinal and the like are used to aid description of the device and need not be seen as limiting on the scope of the invention as claimed.

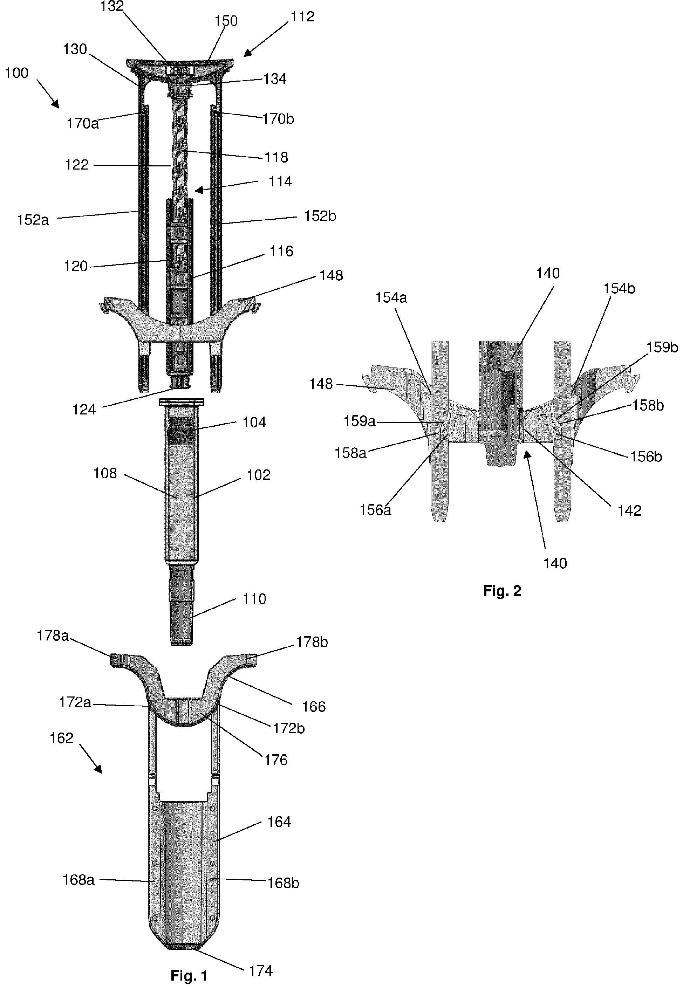

Figure 1 shows an exploded view of an exemplary pre-filled syringe 100. The pre-filled syringe 100 comprises a barrel 102, having an opening (not visible in Figure 1) at a forward end, and a bung 104.

The barrel 102 comprises a medicament 108 located in the volume formed between the bung 104 and the forward opening. The medicament 108 is configured for delivery from the syringe to, for example, a patient.

The opening may be configured to receive a hypodermic needle (covered by a needle shield 110 in Figure 1). The hypodermic needle may be hollow. The opening in the barrel 102 creates a fluid path between the barrel 102 and the hollow channel of the hypodermic needle to allow for delivery of the medicament 108 to a patient via the hypodermic needle.

The pre-filled syringe 100 may further comprise a plunger rod assembly 112. The plunger rod assembly may comprise a plunger rod 114. The plunger rod 114 is configured to be received inside the barrel 102. When the pre-filled syringe 100 is in use by a user, the plunger rod 114 is configured to move within the barrel 102 on an inward stroke and drive the bung 104 forwards such that the medicament 108 is expelled from the forward opening of the barrel 102 by the bung 104 and through the hypodermic needle.

The plunger rod 114 may comprise a first portion (or forward portion) 116, and a second portion (or rearward portion) 118. The first portion 116 and the second portion 118 may be coupled and axially moveable with respect to one another such that the length of the plunger rod 114 can be adjusted before assembly of the pre-filled syringe 100 is completed.

In the exemplary pre-filled syringe 100 of Figure 1, the second portion 118 is telescopically received in the first portion 116. The skilled person will however appreciate that in alternative arrangements, the first portion 116 may be telescopically received in the second portion 118. In other alternative arrangements, the first and second portions may be configured to slide alongside one another. The skilled person will be able to envisage other further configurations.

In the exemplary arrangement shown in Figures 1 and 2, the first portion 116 comprises a first screw thread 120 configured to engage a corresponding second screw thread 122 of the second portion 118. That is, the first and second portions 116, 118 may comprise reciprocal threads 120, 122 such that relative rotation between the first and second portions 116, 118 is allowable. The reciprocal screw threads 120, 122 may be configured to partially translate relative rotation into axial movement of at least one of the first and second portions 116, 118 with respect to the other of the first and second portions 116, 118. In this way, the axial position of one of the first portion 116 and second portion 118 with respect to the other of the first portion 116 and second portion 118 can be altered. The skilled person will understand that in alternative arrangements, the first and second portions could comprise corresponding ramped surfaces configured to allow relative rotation between the first and second portions.

The plunger rod 114 comprises a forward end 124. In the exemplary pre-filled syringe 100 of Figure 1, the forward end 124 of the plunger rod 114 is configured to be separated from the bung 106 when the pre-filled syringe is in an assembled configuration, prior to use of the pre-filled syringe. The forward end 124 of the plunger rod 114 may be separated from the bung 104 by a distance less than or equal to the height of the bung 106. In exemplary pre-filled syringes 100, the forward end 124 of the plunger rod 114 may be separated from the bung 106 by a distance of 2 mm to 4 mm. For example, the forward end 124 of the plunger rod 114 may be separated from the bung by 2 mm, the forward end 124 of the plunger rod 114 may be separated from the bung by 3 mm, and the forward end 124 of the plunger rod 114 may be separated from the bung by 4 mm.

The plunger rod assembly 112 may further comprise a rearward member 130. The second portion 118 may be engageable and disengageable with the rearward member 130. In the exemplary pre-filled syringe 100 of Figure 1, the second portion 118 (that is, the rearward portion) of the plunger rod 114 is engageable and disengageable with the rearward member 130. In exemplary pre-filled syringes, engagement of the second portion 118 and the rearward member 130 may rotationally fix the second portion with respect to the rearward member 130. In some arrangements, engagement of the second portion 118 and the rearward member 130 may rotationally fix the second portion with respect to the first portion 116. When the second portion 118 is disengaged from the rearward member 130, the second portion 118 may be free to rotate with respect to the first portion 116 and/or the rearward member 130.

It is noted that references herein to rotationally fixing features and/or making features free to rotate is, unless otherwise stated, related to fixing features of exemplary devices during assembly and in the assembled state, not during operation of the device.

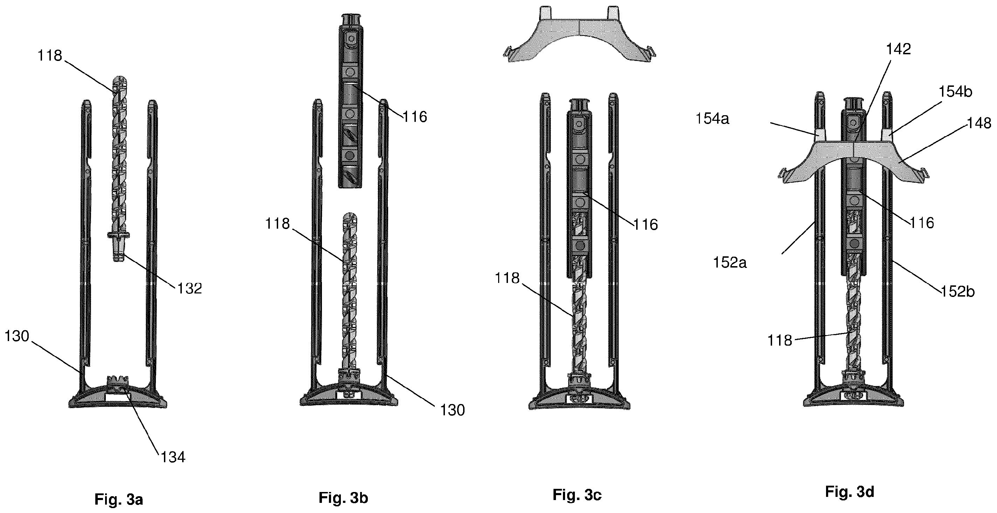

The second portion 118 may comprise at least one rearwardly-extending prong 132 (see also Figure 3a) configured to be received in a corresponding aperture 134 of the rearward member 130. The at least one prong may comprise a barb configured to engage with a surface of the aperture 134 to hold the second portion in engagement with the rearward member 130 and prevent unintended disengagement. The at least one prong 132 may be radially deformable to allow engagement and disengagement of the prong 132 with the surface of the aperture 134 of the rearward member 130. Exemplary pre-filled syringes 100 may comprise a plurality of rearwardly-extending prongs, for example, a pair of opposed rearwardly-extending prongs. As explained in more detail below, the separation between bung 104 and the forward end 124 of the plunger rod 114 may at least partially depend on a length of the at least one prong 132.

The pre-filled syringe 100 may further comprise a rotational lock 140 (visible in Figure 2). The plunger rod 114 may be engageable with the rotational lock 140. The rotational lock 140 may be configured to rotationally fix the first portion 116 relative to the barrel 102 when the first portion 116 is engaged with the rotational lock 140.

In the exemplary pre-filled syringe 100, the rotational lock 140 comprises a keyed aperture 142 (visible in Figure 2) corresponding to at least part of the cross-section of the plunger rod 114. The keyed aperture may be non-circular. In the pre-filled syringe 100 of Figure 1, the cross-section of the first portion (or forward portion) 116 of the plunger rod 114 corresponds to the keyed aperture 142 of the rotational lock 140, such that when the first portion 116 is engaged with the keyed aperture 142, rotation of the first portion is prevented. For example, the cross-section of the first portion (or forward portion) 116 of the plunger rod 114 may correspond to a shape of the keyed aperture 142

The exemplary pre-filled syringe 100 of Figure 1 may further comprise a forward member 148. The forward member 148 may comprise the rotational lock 140. The forward member 148 may be configured to engage a handle portion 166 when the pre-filled syringe 100 is in an assembled configuration.

The forward member 148 may be slidably engaged with the rearward member 130. In the exemplary pre-filled syringe of Figure 1, the rearward member 130 comprises a head 150 and legs 152a, 152b. The forward member 148 may comprise channels 154a, 154b (visible in Figure 2) configured to receive the legs 152a, 152b. The forward member 148 is therefore slideable along the legs 152a, 152b of the rearward member 130 to allow axial movement of the forward member 148 relative to the rearward member 130.

In the exemplary pre-filled syringe 100 of Figure 1, the forward member 148 may comprise at least one latch configured to engage a corresponding retaining recess of the rearward member 130. The exemplary forward member 148 may comprise a pair of latches 156a, 156b (visible in Figure 2) configured to engage corresponding retaining recesses 158a, 158b on the rearward portion 130. The latches 156a, 156b may be located at a point along the channels 154a, 154b of the forward portion 148. The retaining recesses 158a, 158b may be located on the legs 152a, 152b of the rearward portion 130. The position of the retaining recesses 158a, 158b along the legs 152a, 152b may be determined to accommodate different fill volumes of the barrel 102. The retaining recesses of the exemplary arrangement shown in the figures comprise an angled rearward surface 159a, 159b that is arranged to allow the prong and the barb to ride over upon application of a rearward force. In this way, the forward member 148 may be moved rearwards. In some arrangements, the latches 156a, 156b may be resiliently deformable. In such arrangements, the legs 152a, 152b may deform the latches 156a, 156b such that the latches ride over a surface of the legs 152a, 152b when the legs are inserted into the channels 154a, 154b and moved forwards along the channels 154a, 154b. The latches 152a, 152b may engage with the retaining recesses 158a, 158b when the retaining recesses 158a, 158b are in a position adjacent to the latches 156a, 156b (such that the latches are able to return to their original, non-deformed position).

When the latches 156a, 156b are engaged with the retaining recesses 158a, 158b, the rearward member 130 is fixed in relation to the forward portion 148. In exemplary assembled pre-filled syringes, the forward portion 148 may be fixed in relation to the barrel 102. Therefore, in exemplary assembled pre-filled syringes, when the latches 156a, 156b are engaged with the retaining recesses 158a, 158b, the rearward member 130 is fixed with respect to the barrel 102. As such, when the plunger rod 114 is engaged with the rearward member 130 (for example, when the prong 132 is engaged with the aperture 134) and the forward member 148, the plunger rod 114 is axially and rotationally fixed with respect to the barrel 102.

The pre-filled syringe 100 further comprises a forward assembly 162. The forward assembly 162 comprises a sheath 164 configured to cover the hypodermic needle of the pre-filled syringe after the medicament 108 has been dispensed from the barrel 102, and a handle portion 166.

The sheath 164 comprises channels 168a, 168b. The channels 168a, 168b of the sheath 164 correspond to the legs 152a, 152b of the rearward member 130 and are configured to allow travel of the legs 152a, 152b within each channel 168a, 168b. The legs 152a, 152b of the rearward member 130 may be received in the channels 168a, 168b of the sheath 164 when the pre-filled syringe 100 is in an assembled configuration. The legs 152a, 152b may be configured to travel within the channels 168a, 168b of the sheath 164 during use of the pre-filled syringe, for example on an inward stroke of the rearward member 130 and the plunger rod 114.

The legs 152a, 152b of the rearward portion 130 may comprise sheath contact surfaces 170a, 170b configured to engage with the sheath 164 during use of the pre-filled syringe 100, at a point on an inward stroke of the rearward member 130 during use of the pre-filled syringe 100. The sheath contact surfaces may engage with the sheath 164 by directly contacting a surface of the sheath 164. In the exemplary pre-filled syringe 100, the sheath contact surfaces 170a, 170b are located at a rearward position on the legs 152a, 152b of the rearward member 130 and are configured to contact a surface of the sheath 172a, 172b located at a rearward position on the sheath 164 (as shown in Figure 2b). In alternative arrangements the sheath contact surfaces may be located at a forward end of the legs 152a, 152b and configured to contact a surface of the sheath located at a forward position on the sheath 164. For example, the sheath contact surfaces may be the end faces of the legs 152a, 152b.

The sheath 164 is configured to surround an outer wall of the barrel 102, which defines an inner dimension of the sheath 164. The sheath 164 comprises an aperture 174 at a forward end. The aperture 174 may be positioned forward of a shoulder of the barrel 102 when the pre-filled syringe is in an assembled configuration. In the exemplary pre-filled syringe 100, the aperture 174 is of smaller dimension (e.g. diameter) than an inner dimension (e.g. diameter) of the sheath 164. In exemplary safety syringes, the aperture 174 of the sheath 164 may be of a smaller dimension (e.g. diameter) than the outer wall of the barrel 102.

The exemplary sheath 164 comprises a lip (not visible in Figure 1). The lip extends radially inwards from an inner wall of the sheath 164 to define the aperture 174. The lip may extend inwards to a point such that the diameter of the aperture 174 is of smaller diameter than the outer wall of the barrel 102. The lip may be configured to contact the shoulder of barrel 102 in an assembled configuration. The lip may be configured to contact a forward surface of the shoulder of the barrel 102.

In alternative arrangements, the diameter aperture 174 of the sheath 164 may be larger than the outer wall of the barrel 102. In such arrangements, the aperture 174 may be positioned at a point along the length of the barrel 102 when the pre-filled syringe is in an assembled state.

The handle portion 166 comprises a main body 176 and flanges 178a, 178b extending laterally from the main body 176. The main body 176 comprises a portion that surrounds the barrel 102 and is fixed thereto. The flanges 178a, 178b are configured to receive the index finger and middle finger of a user while the thumb applies a force to the head 150 of the rearward member 130 when using the pre-filled syringe, although any combination of fingers and/or thumb could be used. The handle portion 166 is fixed to the barrel 102 by an aperture configured to receive an end of the barrel 102 at a distal end to the opening coupled to the needle. In the embodiment of Figure 1, the barrel 102 comprises a radially protruding lip that is received by the handle portion 116, however in alternative arrangements, the handle portion 116 may receive syringe barrels without radially protruding lips.

The sheath 164 may be at least partially received within the main body 176 of the handle portion 166.

The handle portion 166 may comprise channels corresponding to the legs 152a, 152b of the rearward member 130, such that the legs 152a, 152b are able to pass through the handle portion 166. In this way, the rearward portion 130 may move in its stroke relative to the handle portion 166 and, therefore, the barrel 102.

The pre-filled syringe 100 may comprise a coupling arrangement configured to couple the rearward member 130 and the plunger rod 114 such that movement of the rearward member 130 on an inward stroke causes an inward stroke of the plunger rod 114. The pre-filled syringe 100 may comprise a decoupling arrangement configured to decouple the rearward member 130 and the plunger rod 114, such that relative movement of the rearward member 130 with respect to the plunger rod 114 is possible.

The inventors have realised that there exists a need to provide a method of assembling a pre-filled syringe such that a separation is formed between the bung and the forward end of the plunger rod. Further, there exists a need to provide a method of assembly of a pre-filled syringe that enables a consistent separation between the bung and the forward end of the plunger rod even when there are variations in the volume of medicament contained in the barrel (and therefore variations in the axial position of the bung within the barrel).

A method of assembling the pre-filled syringe 100 is described below with reference to Figures 3a-i. Exemplary methods comprise setting the length of the plunger rod 114 and fitting the plunger rod assembly 112 to the barrel 102. As described below, setting the length of the plunger rod 114 may comprise partially inserting the plunger rod 114 into the barrel 102 such that the forward end of the plunger rod 114 contacts the bung 104.

As shown in Figure 3a, exemplary methods of assembly of the pre-filled syringe 100 comprise engaging the rearward portion 118 of the plunger rod 114 with the rearward member 130. Engaging the rearward portion 118 with the rearward member 130 rotationally fixes the rearward portion 118 with respect to the rearward member 130. In the exemplary plunger rod assembly 112, engaging the rearward portion of the plunger rod 114 comprises engaging the at least one prong 132 with the aperture 134 of the rearward member 130.

As shown in Figure 3b, the forward portion 116 of the plunger rod 114 may then be engaged with the rearward portion 118. Specifically, the forward portion 116 may be moved into a position such that the rearward portion 118 is telescopically received by the forward portion 116. Engagement of the forward portion 116 and the rearward portion 118 causes engagement of the reciprocal screw threads 120, 122 of the rearward portion 118 and the forward portion 116.

A rotational force (or axial force) may then be applied to the forward portion 116 in order to position the forward portion 116 with respect to the rearward portion 118. The reciprocal screw threads 120, 122 may partially translate the applied rotational force into an axial force (or axial force into a rotational force) such that the forward portion 116 moves axially relative to the rearward portion 118 (which is rotationally fixed as described above). This allows the axial position of the forward portion 116 with respect to the rearward portion 118 to be altered (and therefore the length of the plunger rod 114 to be altered).

Once the first portion 116 has been coupled to the second portion 118 by rotating the forward portion 116 with respect to the rearward portion 118, the method may further comprise rotationally fixing the forward portion 116 with respect to the barrel and/or the remainder of the plunger rod assembly. This may prevent the plunger rod length being further altered, at least whilst the second portion 118 is rotationally fixed with respect to the remainder of the plunger rod assembly. The initial length of the plunger rod 114 may be different to the assembled length of the plunger rod 114 (that is, the length of the plunger rod 114 when the pre-filled syringe 100 is in an assembled configuration).

Rotationally fixing the forward portion 116 with respect to barrel 104 and/or the remainder of the plunger rod assembly may comprise engaging the forward portion 116 with the rotational lock 140, as shown in Figures 3c and 3d.

Engaging the forward portion 116 with the rotational lock 140 comprises engaging the forward member 148 with the forward portion 116 of the plunger rod 114. The channels 154a, 154b of the forward member 148 may receive the legs 152a, 152b of the rearward member 130. The keyed aperture 142 of the forward member 148 may receive the forward portion 116, thereby rotationally fixing the first portion 116 with respect to the barrel and the remainder of the plunger rod assembly.

The forward member 148 may then be axially moved with respect to the rearward member 130 and the forward portion 116 of the plunger rod 114 until the latches 156a, 156b of the forward member 148 engage with the retaining recesses 158a, 158b of the legs 152a, 152b of the rearward member 130. For example, the forward member 130 may be axially moved along the legs 152a, 152b of the rearward member. Once the latches 156a, 156b and the retaining recesses 158a, 158b are engaged, the axial position of the forward member 148 with respect to the plunger rod 114 and the rearward member 130 is fixed. Further, because the cross-section of the forward portion 116 of the plunger rod 114 corresponds to the keyed aperture 142 of the rotational lock 140, as described above, rotation of the forward portion 116 with respect to the barrel and/or the remainder of the plunger rod assembly is prevented.

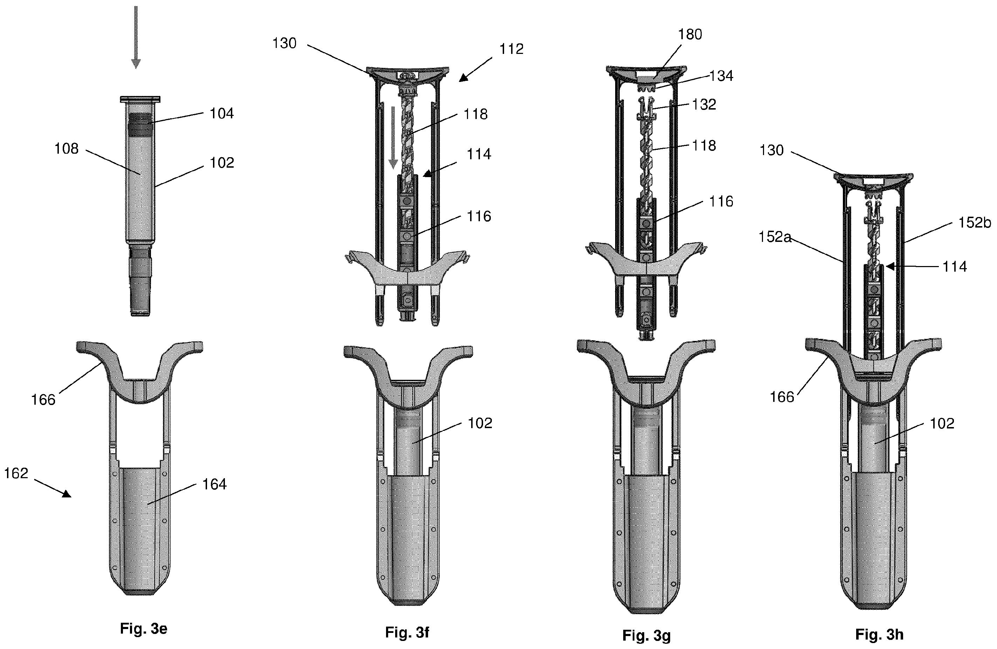

As shown in Figure 3e, the barrel 102 comprising the bung 104, the forward opening and the medicament 108 may be inserted into the forward assembly 162.

The plunger rod assembly 112 may then be fitted to the barrel 102 of the pre-filled syringe 100.

As shown in Figure 3f, prior to fitting the plunger rod assembly 112 to the barrel 102, the rearward portion 118 of the plunger rod 114 is disengaged from the rearward member 130. Disengaging the rearward portion 118 from the rearward member 130 may comprise applying a forward axial force to the rearward portion 118 to disengage the at least one prong 132 from the recess 134 of the rearward member 130.

Disengaging the rearward portion 118 from the rearward member 130 rotationally disengages the rearward portion 118 from the rearward member 130. That is, disengaging the rearward portion 118 from the rearward member allows rotation of the rearward portion 118 with respect to the rearward member. In addition, because the forward member is rotationally fixed by the rotational lock 140, the second portion 118 is free to rotate with respect to the forward portion 116.

Disengaging the rearward portion 118 further allows axial movement of the forward portion 116 within the keyed aperture (although the forward portion 116 is still rotationally fixed with respect to the rearward portion 118). That is, disengaging the rearward portion 118 axially decouples the plunger rod 114 from the rearward member 130, such that relative axial movement between the plunger rod 114 and the rearward member 130 is possible.

The method of assembling the pre-filled syringe may further comprise preventing rotational re-engagement of the rearward portion 118 with the rearward member 130 (see Figure 3g). As will be described below, this allows relative rotation between the first portion 116 and the second portion 118 in order to set the length of the plunger rod.

In exemplary methods and apparatus, preventing re-engagement of the rearward portion 118 and the rearward member 130 may comprise preventing re-insertion of the at least one prong 132 into the aperture 134 of the rearward member 130. For example, the method of assembly may further comprise obscuring the aperture 134 of the rearward member by inserting a stopper 180 into the aperture 134, to prevent the at least one prong 132 of the rearward portion 118 from being re-engaged with the aperture 134. This prevents the rearward portion 118 from engaging with the rearward member 130 when the plunger rod 114 is moved rearward during further assembly steps (as explained below).

The plunger rod assembly 112 may then be fitted to the barrel 102. Fitting the plunger rod assembly 112 to the barrel 102 may comprise at least partially inserting the plunger rod 114 into the barrel 102 such that the forward end 124 of the plunger rod 114 contacts the bung 104. Further insertion of the plunger rod into the barrel results in a rearward axial force on the first portion 116 for setting the length of the plunger rod.

Fitting the plunger rod assembly 112 to the barrel 102 may further comprise engaging the legs 152a, 152b of the rearward member 130 with the handle portion 166 and the barrel 102, such that the rearward assembly 130 is slideable with respect to the barrel 102. Specifically, the legs 152a, 152b may be received within channels of the handle portion 166 such that the legs 152a, 152b are slideable within the channels of the handle portion 166 to allow axial movement of the rearward member 130 with respect to the barrel 102.

Once the plunger rod 114 has been inserted into the barrel 102, the plunger rod assembly 112 may be moved forward towards the bung 104.

Moving the plunger rod assembly 112 towards the bung 104 moves the plunger rod 114 further into the barrel 104. This results in the forward end 124 of the plunger rod 114 contacting the bung 104. The point at which the forward end 124 of the plunger rod 114 contacts the bung 104 may depend on the amount of medicament contained within the barrel 102, and therefore the axial position of the bung 104 within the barrel 102. In exemplary methods, the initial length of the plunger rod 114 is set (as described above and shown in Figure 3d) such that the forward end 124 of the plunger rod 114 contacts the bung 104 during insertion of the plunger rod 114 within the barrel 102 before the plunger rod assembly 112 is fitted to the barrel. That is, before the forward member 148 engages with the handle portion 166.

Exemplary methods of assembly comprise moving the plunger rod assembly 112 further forwards after the forward end 124 of the plunger rod 114 contacts the bung 104. Because the plunger rod 114 is rotationally decoupled from the rearward member 130 (as described above), further forward movement of the rearward member 130 results in a rearward axial force on the first portion 116 and therefore the second portion 118. The second portion 118 moves to meet the rearward member 130. Specifically, the rearward member 130 is moved further forward until the at least one prong 132 contacts the rearward member 130.

As described above, because the aperture 134 of the rearward member 130 is obstructed, for example by the stopper 170, the at least one prong 132 is unable to re-engage with the aperture 134.

Once the rearward member 130 contacts the rearward portion 118 of the plunger rod 114, further forward movement of the rearward member 130 exerts an axial force on the first portion 116 and the rearward portion 118 of the plunger rod 114. Because further forward movement of the forward portion 116 of the plunger rod 114 is resisted by the medicament 108 contained within the barrel 102 (since the forward end 124 of the plunger rod 114 is in contact with the bung 104, which in turn is in contact with the medicament 108), the axial force causes the rearward portion 118 to rotate due to the reciprocal threads 120, 122 on the forward portion 116 and the rearward portion 118. This rotation is partially translated into axial movement by the reciprocal threads 120, 122 and as such, the axial position of the rearward portion 118 with respect to the forward portion 116 is altered. Further forward movement of the rearward member 130 therefore acts to rotate the rearward portion 118 with respect to the forward portion 116 reduce the length of the plunger rod 114.

The further forward movement of the rearward member 130 (and therefore the axial movement of the rearward portion 118 with respect to the forward portion 116) continues until the plunger rod assembly 112 is fitted to the barrel 102. The plunger rod assembly may be fitted to the barrel when the forward member 148 engages with the handle portion 166. This is shown in Figure 3h. The length of the plunger rod 114 is then set.

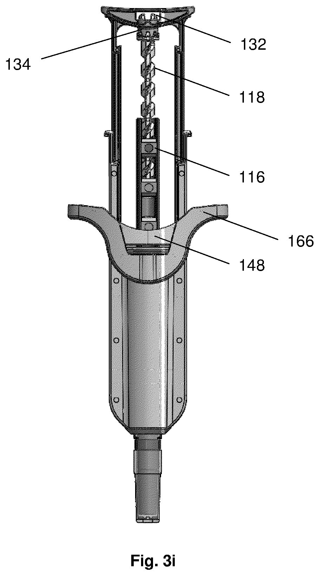

The stopper 180 may be removed from the aperture 134 of the rearward member 130. The plunger rod 114 is then moved rearward, away from the bung 104, such that the rearward portion 118 of the plunger rod 114 re-engages with the rearward member 130, as shown in Figure 3i. Because the length of the plunger rod 114 remains constant during this rearward movement, the rearward movement of the plunger rod 114 causes a separation between the forward end 124 of the plunger rod 114 and the bung 104. The separation may correspond to the length of the at least one prong 132.

The separation can be predetermined, for example, by adjusting the length of the prongs. For example, if the prongs are 2 mm in length, moving the plunger rod rearwards to engage the prongs with the aperture may result in a rearward movement of the plunger rod of 2 mm, providing a separation of 2 mm between the forward end of the plunger rod and the bung. As such, a consistent separation between the bung and the forward end of the plunger rod when the pre-filled syringe is in an assembled configuration can be achieved regardless of minor variations in fill volumes.

Re-engaging the plunger rod 114 and the rearward member 130 rotationally fixes the rearward portion 118 with respect to the remainder of the plunger rod assembly, and therefore the forward portion 116. As such, once the plunger rod 114 is re-engaged with the rearward member 130, further relative movement of the forward portion and rearward portion is not possible (until the syringe is used). Further, re-engaging the plunger rod 114 with the rearward member 130 axially coupled the plunger rod 114 and the rearward member 130 such that relative axial movement therebetween is not possible in the assembled configuration.

An alternative method of assembling a pre-filled syringe 100 to provide a gap between the bung and the forward end of the plunger rod is described below. The alternative method may comprise setting a length of the plunger rod 114 before fitting the plunger rod assembly to the barrel 102. Setting the length of the plunger rod 114 may comprise rotating the forward portion 116 with respect to the rearward portion 118 before fitting the plunger rod assembly 112 to the barrel 102. The reciprocal threads 120, 122 of the forward portion 116 and the rearward portion 118 partially translate the rotational force into an axial force to allow the forward portion 116 to be positioned with respect to the rearward portion 118. Once the length of the plunger rod 114 has been set, the forward portion 116 may be rotationally fixed with respect to the rearward portion 118. The plunger rod assembly 112 may then be fitted to the barrel 102 without further adjustment of the length of the plunger rod 114.

The method may comprise assembling the plunger rod assembly 112 as described above in respect of Figures 3a to 3c.

The skilled person will appreciate that the assembled length (that is, the length of the plunger rod 114 desired in the assembled pre-filled syringe) of the plunger rod could be set in various ways. For example, the assembled length of the plunger rod 114 may be set by rotating the forward portion 116 with respect to the rearward portion 118 until the assembled length is reached. Once the assembled length is reached, the forward portion 116 may be engaged with the rotational lock 140 to rotationally fix the forward portion 116 with respect to the rearward portion 118, and therefore fix the length of the plunger rod 114.

Alternatively, in arrangements in which the rotational lock 140 comprises the keyed aperture and forms part of the forward member 148, the forward member may be brought into engagement with the plunger rod 114 before the assembled length of the plunger rod is set. That is, the channels 154a, 154b of the forward member 148 may receive the legs 152a, 152b of the rearward member 130. The keyed aperture 142 of the forward member 148 may receive the forward portion 116.

The forward member 148 may then be moved rearwards, along the legs 152a, 152b of the rearward member 130, towards the head 150 of the rearward member 130 until the keyed aperture 142 of the rotational lock 140 disengages from the forward portion 116 of the plunger rod 114. Once the rotational lock 140 has disengaged from the forward portion 116 of the plunger rod 114, the forward portion 116 is free to rotate relative to the rearward portion 118. The assembled length of the plunger rod can then be set by rotating the forward portion 116 with respect to the rearward portion 118 as described above.

Once the assembled length of the plunger rod 114 has been set, the forward member 148 may be moved forwards along the legs 152a, 152b of the rearward member 130 until the rotational lock 140 engages the forward portion 116 of the plunger rod 114. Specifically, the forward member 148 may be moved along the legs 152a, 152b until the keyed aperture 142 engages with the forward portion 116 of the plunger rod 114.

The forward member 148 may be moved forwards along the legs 152a, 152b of the rearward member 130 until the latches 156a, 156b of the forward member 148 engage with the retaining recesses 158a, 158b of the legs 152a, 152b of the rearward member 130. Once the latches 156a, 156b and the retaining recesses 158a, 158b are engaged, the axial position of the forward member 148 with respect to the plunger rod 114 and the rearward member 130 is fixed. Further, because the cross-section of the forward portion 116 of the plunger rod 114 corresponds to the keyed aperture 142 of the rotational lock 140, as described above, rotation of the forward portion 116 with respect to the rearward portion 118 is prevented. As such, the length of the plunger rod 114 is set to the assembled length.

The plunger rod assembly 112 may then be fitted to the barrel 102. Fitting the plunger rod assembly 112 to the barrel 102 may comprise inserting the plunger rod 114 into the barrel.

Fitting the plunger rod assembly 112 to the barrel 102 may further comprise engaging the legs 152a, 152b of the rearward member 130 with the barrel 102, such that the rearward member 130 is slideable with respect to the barrel 102. For example, the legs 152a, 152b of the rearward member 130 may be engaged with the handle portion 166. Specifically, the legs 152a, 152b may be received within channels of the handle portion 166 such that the legs 152a, 152b are slideable within the channels of the handle portion 166.

Once the plunger rod 114 has been inserted into the barrel 102, the plunger rod assembly 112 may be moved forward towards the bung 104.

Moving the plunger rod assembly 112 towards the bung 104 moves the plunger rod 114 further into the barrel 102. The plunger rod assembly 112 may be moved towards the bung 104 until the plunger rod assembly 112 is fitted to the barrel 102. For example, the plunger rod assembly 112 may be moved towards the bung 104 until the forward member 148 engages with the handle portion 166. Because the length of the plunger rod 114 is set to the assembled length before the plunger rod assembly 112 is fitted to the barrel 102, the forward end 124 of the plunger rod 114 does not contact the bung 102 during assembly. As such, when the plunger rod assembly 112 is fitted to the barrel 102, the forward end 124 of the plunger rod 114 is separated from the bung 104.

In another arrangement, the plunger rod may be inserted into the barrel while the rotational lock 140 is disengaged from the first portion 116. This will allow rotation of the first portion 116 as the forward end of the first portion contacts the bung. After the plunger rod assembly has been fitted to the barrel, the first portion 116 may be rotated a predetermined number of times in order to set the amount of separation between the forward end of the plunger rod and the bung. The rotational lock may be engaged with the first portion 116 to set the length of the plunger rod.

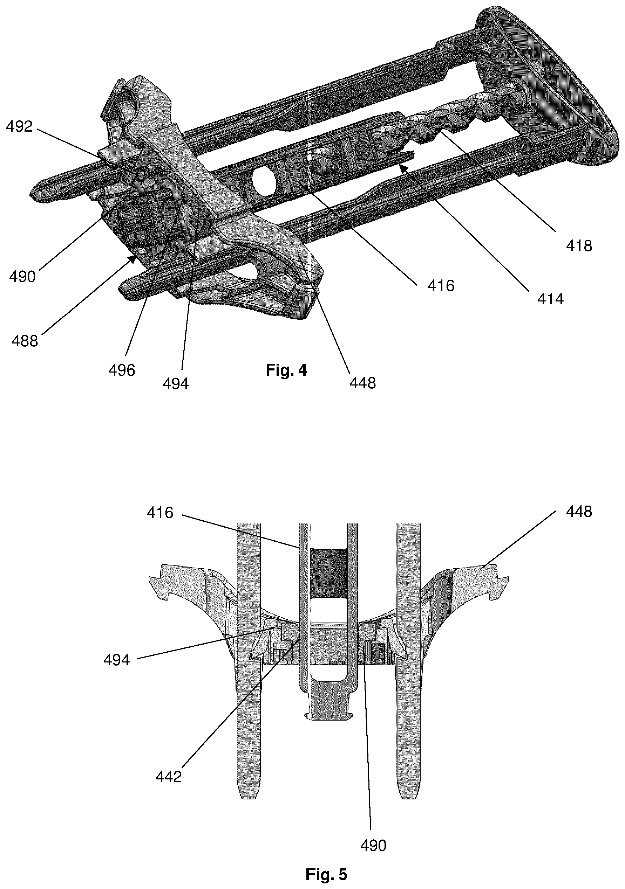

Figure 4 shows an alternative pre-filled syringe 400. Many of the features of the pre-filled syringe 400 are similar to the features of the pre-filled syringe 100 of Figure 1, and as such, a description of these features is not given again here. Corresponding reference numerals are used to identify the similar features.

The pre-filled syringe 400 comprises a ratchet 488 configured for engagement with the plunger rod 414. The ratchet 488 is one example of a unidirectional rotational lock and there may be other implementations of this. The ratchet 488 may be configured for engagement with the first portion 416 of the plunger rod 414. The ratchet 488 may be configured to prevent rotation of the first portion 416 in a first direction, and to allow rotation of the first portion 416 in a second direction (opposite to the first direction).

The exemplary ratchet 488 shown in Figure 4 comprises a wheel 490 comprising teeth 492. The ratchet 488 may further comprise a recessed portion 494 configured to receive the wheel 490 and comprising corresponding teeth 496. The teeth 492 of the wheel 490 and/or the teeth 496 of the recessed portion 494 may be shaped such that rotation of the wheel 490 in the second direction is possible (since the teeth of the wheel 490 are able to pass over the corresponding teeth 496 of the recessed portion 494). The teeth 492 of the wheel 490 and/or the teeth 496 of the recessed portion 494 may be shaped such that rotation of the wheel 490 in the first direction results in the engagement of the teeth 492 and the corresponding teeth 496 such that rotation of the wheel 490 in the first direction is prevented. In this example, since the rearward portion 418 is rotationally fixed relative to the rearward member 430, rotation of the forward portion 416 results in axial movement of forward portion 416, as described below. The axial position of the forward portion 416 with respect to the rearward portion 418 may be adjusted in intervals using the ratchet 488.

The wheel 490 may be engaged with the first portion 416 such that they rotate together. For example, the wheel 490 may comprise a keyed aperture 442 (see Figure 5) corresponding to the cross-section of the first portion. In the pre-filled syringe 400 of Figure 4, the cross-section of the first portion (or forward portion) 416 of the plunger rod 414 corresponds to the keyed aperture 442 of the wheel 490, such that when the first portion 416 is engaged with the keyed aperture 442, rotation of the first portion 416 relative to the wheel 490 is prevented.

In the exemplary pre-filled syringe 400 of Figure 4, the forward member 448 may comprise the recessed portion 494. As such, the wheel 490 may be received by the forward member 448 and the forward portion 416 of the plunger rod 414 may be received by the wheel 490 (see Figure 5).

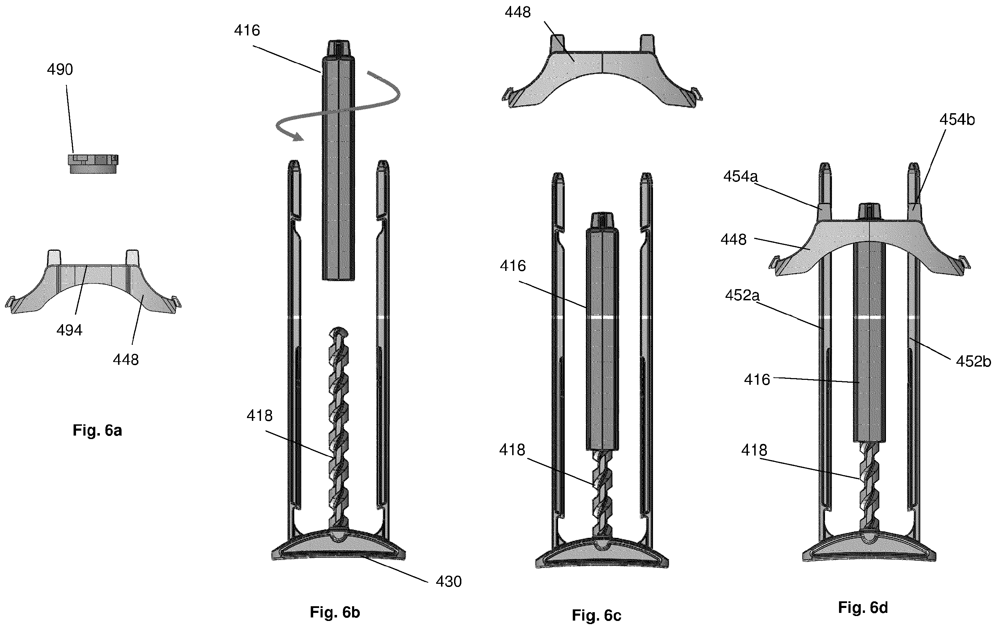

A method of assembling the pre-filled syringe 400 is described below with reference to Figures 6a - g.

As shown in Figure 6a, the wheel 490 is inserted into the recessed portion 494 of the forward member 448.

In the exemplary pre-filled syringe 400, the rearward portion 418 of the plunger rod 414 is integral with the rearward member 130. That is, the rearward portion 418 of the plunger rod 414 cannot be disengaged from the rearward member 130. In alternative arrangements, the rearward portion may be engageable with and disengageable from the rearward member. In such arrangements, the rearward portion may be engaged with the rearward member. In either arrangement, the rearward portion 418 is rotationally fixed with respect to the rearward member 430.

As show in Figure 6b, the forward portion 416 of the plunger rod 414 may then be engaged with the rearward portion 418. Specifically, the forward portion 416 may be moved into a position such that the rearward portion 418 is telescopically received by the forward portion 416. Engagement of the forward portion 416 and the rearward portion 418 causes engagement of the reciprocal screw threads 420, 422 of the rearward portion 418 and the forward portion 416.

A rotational force (or axial force) may then be applied to the forward portion 416 in order to position the forward portion 116 with respect to the rearward portion 418. The reciprocal screw threads 420, 422 may partially translate the applied rotational force into an axial force such that the forward portion 416 moves axially relative to the rearward portion 418 (which is rotationally fixed as described above). This allows the axial position of the forward portion 416 with respect to the rearward portion 418 to be altered (and therefore the length of the plunger rod 414 to be altered), as shown in Figure 6c.

Once an initial length of the plunger rod 414 has been set, by rotating the forward portion 416 with respect to the rearward portion 418, the method may further comprise engaging the forward portion 416 with the ratchet 488. The initial length of the plunger rod 414 may be different to the assembled length of the plunger rod 114 (that is, the length of the plunger rod 114 when the pre-filled syringe 100 is in an assembled configuration). Specifically, the initial length of the plunger rod 414 may be such that when the plunger rod assembly 412 is fitted to the barrel 402, the plunger rod 414 does not contact the bung 404.

Engaging the forward portion 416 with the ratchet 488 may comprise inserting the forward portion 416 into the keyed aperture 442 of the wheel 490 of the ratchet 488. The rearward member 430 may be engaged with the forward member 448. Engaging the rearward member 430 with the forward member 448 may comprise inserting the legs 452a, 452b of the rearward member 430 into the channels of 454a, 454b of the forward member 448.

The forward member 448 may then be axially moved with respect to the rearward member 430 and the forward portion 416 of the plunger rod 414 until the latches 456a, 456b of the forward member 448 engage with the retaining recesses 458a, 458b of the legs 452a, 452b of the rearward member 430. For example, the forward member 430 may be axially moved along the legs 452a, 452b of the rearward member. Once the latches 456a, 456b and the retaining recesses 458a, 458b are engaged, the axial position of the forward member 448 with respect to the plunger rod 414 and the rearward member 430 is fixed.

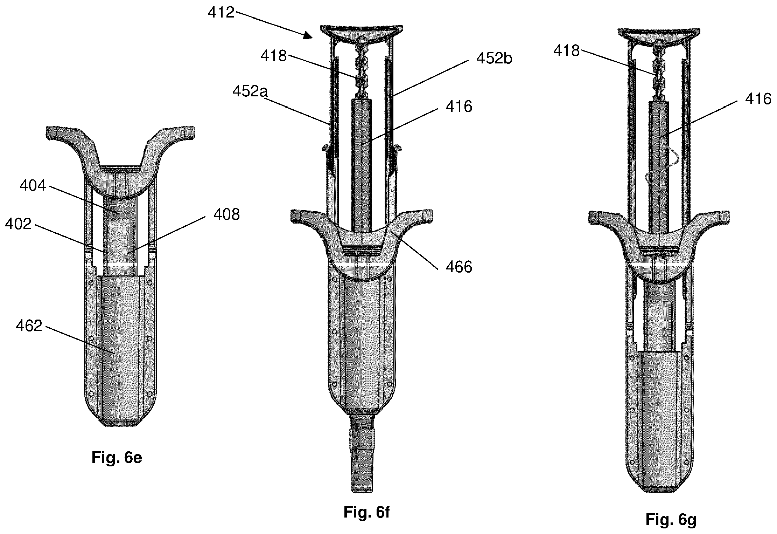

As shown in Figure 6e, the barrel 402 comprising the bung 404, the opening and the medicament 408 may be inserted into the forward assembly 462.

The plunger rod assembly 412 may then be fitted to the barrel 402, as shown in Figure 6f. Fitting the plunger rod assembly 412 to the barrel 402 may comprise inserting the plunger rod 414 into the barrel.

Fitting the plunger rod assembly 412 to the barrel 402 may further comprise engaging the legs 452a, 452b of the rearward member 430 with the barrel 402, such that the rearward member 430 is slideable with respect to the barrel 402. For example, the legs 452a, 452b of the rearward member 430 may be engaged with the handle portion 466. Specifically, the legs 452a, 452b may be received within channels of the handle portion 466 such that the legs 452a, 452b are slideable within the channels of the handle portion 466.

Once the plunger rod 414 has been inserted into the barrel 402, the plunger rod assembly 412 may be moved forward towards the bung 404.

Moving the plunger rod assembly 412 towards the bung 404 moves the plunger rod 414 further into the barrel 402. The plunger rod assembly 412 may be moved towards the bung 404 until the plunger rod assembly 412 is fitted to the barrel 402. For example, the plunger rod assembly 412 may be moved towards the bung 404 until the forward member 448 engages with the handle portion 466. Because the length of the plunger rod 414 is set to the assembled length before the plunger rod assembly 412 is fitted to the barrel 402, the forward end 424 of the plunger rod 414 does not contact the bung 404 during assembly.

Once the plunger rod assembly 412 has been fitted to the barrel 402, a rotational force may be applied to the forward portion 416 (see Figure 6g). A rotational force may be applied to the forward portion 416 in the second direction. This causes the forward portion 416 to rotate with respect to the rearward portion 418. Due to the reciprocal threads 420, 422 on the forward portion 416 and the rearward portion 418, the rotational force is translated into axial movement of the forward portion 416 with respect to the rearward portion 418. Rotation of the forward portion 416 with respect to the rearward portion 418 in the second direction is possible due to the ratchet 488.

The forward portion 416 is rotated with respect to the rearward portion 418 until a desired separation between the forward end 424 of the plunger rod 414 is reached.

It is noted that many of the features of the exemplary apparatus described above and shown in the drawings may be included in other exemplary apparatus. As such, the different drawings are not necessarily to be considered as separate embodiments and features from one drawing may be transferred to an apparatus in another drawing. It is also noted that any of the features of the pre-filled syringes described herein may be used in a safety syringe apparatus for fitting to a syringe.

The skilled person will be able to envisage other pre-filled syringes and features thereof without departing from the scope of the appended claims. In particular, it is noted that one or more features included in one or more drawings may be integrated pre-filled syringes shown in other drawings, as will be appreciated by the skilled person.

## Claims

1. A method of assembling a pre-filled syringe (100) comprising:
setting a length of a plunger rod (114) of a plunger rod assembly (112); and
fitting the plunger rod assembly (112) to a barrel (102) of the syringe (100), the barrel (102) having an opening at a forward end thereof and comprising a bung (104) positioned therein and creating a volume between the bung (104) and the opening, the volume at least partially filled with a substance for delivery from the syringe (100),
wherein setting the length of the plunger rod assembly (112) comprises at least partially inserting the plunger rod (114) into the barrel (102) such that a forward end (124) of the plunger rod (114) contacts the bung (104),
**characterised in that** the method further comprises moving the plunger rod (114) rearward after setting the length of the plunger rod (114) and after the forward end (124) of the plunger rod (114) contacts the bung (104) for defining a separation between the bung (104) and the forward end (124) of the plunger rod (114) , such that, after assembly, the length of the plunger rod (114) defines a separation between the bung (104) and the forward end (124) of the plunger rod (114).

2. A method according to claim 1, wherein setting the length of the plunger rod (114) comprises positioning a first portion (116) of the plunger rod (114) with respect to a second portion (118) of the plunger rod (114).

3. A method according to claim 2, wherein the first and second portions (116, 118) of the plunger rod (114) are coupled and the first portion (116) is axially moveable with respect to the second portion (118),
and wherein positioning the first portion (116) with respect to the second portion (118) comprises axially moving the first portion (116) with respect to the second portion (118).

4. A method according to any preceding claim, comprising further insertion of the plunger rod (114) into the barrel (102), wherein the further insertion results in an axial force acting on the first portion (116) for positioning the first portion (116) with respect to the second portion (118), and optionally wherein one of the first and second portions (116, 118) is telescopically received in the other of the first and second portions (116, 118).

5. A method according to any of claims 2 to 4, wherein setting the length of the plunger rod (114) comprises rotating at least one of the first portion (116) and second portion (118) with respect to the other of the first portion (116) and the second portion (118), and optionally wherein setting the length of the plunger rod (114) comprises engaging reciprocal threads (120, 122) of the first portion (116) and the second portion (118) for translating rotational force into axial force and vice-versa, and rotating one of the first portion (116) and second portion (118) with respect to the other of the first portion (116) and the second portion (118).

6. A method according to claim 5 when dependent directly or indirectly on claim 4, wherein the first portion (116) is a forward portion and the second portion (118) is a rearward portion, the method further comprising:
engaging the first portion (116) with a rotational lock (140) to rotationally fix the first portion (116) relative to the barrel (102) before further insertion of the plunger rod (114) into the barrel (102), such that the second portion (118) rotates relative to the first portion (116).

7. A method according to claim 6, wherein the rotational lock (140) comprises a keyed aperture (142), a cross section of at least part of the plunger rod (114) corresponding to a shape of the keyed aperture (142), and/or the method further comprising rotationally fixing the second portion (118) with respect to the first portion (116) after fitting the plunger rod assembly (112) to the barrel (102).

8. A method according to claim 7, wherein the plunger rod assembly (112) further comprises a rearward member (130) and at least one leg (152a, 152b), the at least one leg (152a, 152b) slidably engaged, directly or indirectly, with the barrel (102) after fitting of the plunger rod assembly (112) to the barrel (102),
and wherein rotationally fixing the second portion (118) with respect to the first portion (116) comprises engaging the second portion (118) with the rearward member (130).

9. A method according to claim 8 when dependent directly or indirectly on claim 6 or 7, the plunger rod assembly (112) further comprising a forward member (148) for fitting to the barrel (102) and slidably engaged with the at least one leg (152a, 152b), the forward member (148) comprising the rotational lock (140).

10. A method according to claim 8 or 9 when dependent on claim 7, wherein engaging the second portion (118) with the rearward member (130) comprises moving the plunger rod (114) rearward.

11. A method according to claim 10, wherein the rearward member (130) comprises at least one aperture (134) for receiving at least one rearwardly extending prong (132) of the second portion (118), and wherein engaging the second portion (118) with the rearward member (130) comprises inserting the at least one prong (132) into the at least one aperture (134), and optionally the method further comprising preventing insertion of the at least one prong (132) into the at least one aperture (134) during further insertion of the plunger rod (114) into the barrel (102).

12. A method according to claim 11, wherein preventing insertion of the at least one prong (132) into the at least one aperture (134) comprises obscuring the at least one aperture (134) and optionally wherein obscuring the at least one aperture (134) comprises inserting a stopper (180) into the at least one aperture (134) to prevent the at least one prong (132) of the rearward portion (130) from being inserted into the at least one aperture (134), and optionally, further comprising removing the stopper (180) from the at least one aperture (134) after fitting the plunger rod assembly (112) to the barrel (102) to allow receipt of the at least one prong (132) within the at least one aperture (134).

## Patentansprüche

1. Verfahren zum Zusammenbauen einer Fertigspritze (100), umfassend:
Einstellen einer Länge einer Kolbenstange (114) einer Kolbenstangenbaugruppe (112); und
Anbringen der Kolbenstangenbaugruppe (112) an einem Zylinder (102) der Spritze (100), wobei der Zylinder (102) an einem vorderen Ende davon eine Öffnung aufweist und einen Stöpsel (104) umfasst, der darin positioniert ist und ein Volumen zwischen dem Stöpsel (104) und der Öffnung erzeugt, wobei das Volumen mindestens teilweise mit einer Substanz zur Abgabe aus der Spritze (100) gefüllt ist,
wobei Einstellen der Länge der Kolbenstangenbaugruppe (112) mindestens teilweise Einführen der Kolbenstange (114) in den Zylinder (102) umfasst, sodass ein vorderes Ende (124) der Kolbenstange (114) den Stöpsel (104) berührt,
**dadurch gekennzeichnet, dass** das Verfahren weiter Bewegen der Kolbenstange (114) nach hinten umfasst, nachdem die Länge der Kolbenstange (114) eingestellt wurde und nachdem das vordere Ende (124) der Kolbenstange (114) den Stöpsel (104) berührt, um einen Abstand zwischen dem Stöpsel (104) und dem vorderen Ende (124) der Kolbenstange (114) zu definieren, sodass die Länge der Kolbenstange (114) nach der Baugruppe einen Abstand zwischen dem Stöpsel (104) und dem vorderen Ende (124) der Kolbenstange (114) definiert.

2. Verfahren nach Anspruch 1, wobei Einstellen der Länge der Kolbenstange (114) Positionieren eines ersten Abschnitts (116) der Kolbenstange (114) in Bezug auf einen zweiten Abschnitt (118) der Kolbenstange (114) umfasst.

3. Verfahren nach Anspruch 2, wobei der erste und zweite Abschnitt (116, 118) der Kolbenstange (114) gekoppelt sind und der erste Abschnitt (116) in Bezug auf den zweiten Abschnitt (118) axial beweglich ist,
und wobei Positionieren des ersten Abschnitts (116) in Bezug auf den zweiten Abschnitt (118) axiales Bewegen des ersten Abschnitts (116) in Bezug auf den zweiten Abschnitt (118) umfasst.

4. Verfahren nach einem vorstehenden Anspruch, umfassend weitere Einführung der Kolbenstange (114) in den Zylinder (102), wobei die weitere Einführung in einer axialen Kraft resultiert, die auf den ersten Abschnitt (116) wirkt, um den ersten Abschnitt (116) in Bezug auf den zweiten Abschnitt (118) zu positionieren, und wobei optional einer des ersten und zweiten Abschnitts (116, 118) teleskopartig in dem anderen des ersten und zweiten Abschnitts (116, 118) aufgenommen ist.

5. Verfahren nach einem der Ansprüche 2 bis 4, wobei Einstellen der Länge der Kolbenstange (114) Drehen mindestens eines des ersten Abschnitts (116) und zweiten Abschnitts (118) in Bezug auf den anderen des ersten Abschnitts (116) und des zweiten Abschnitts (118) umfasst, und wobei optional Einstellen der Länge der Kolbenstange (114) In-Eingriff-Bringen gegenläufiger Gewinde (120, 122) des ersten Abschnitts (116) und des zweiten Abschnitts (118) zum Umwandeln von Drehkraft in Axialkraft und umgekehrt und Drehen eines des ersten Abschnitts (116) und des zweiten Abschnitts (118) in Bezug auf den anderen des ersten Abschnitts (116) und des zweiten Abschnitts (118) umfasst.

6. Verfahren nach Anspruch 5, wenn direkt oder indirekt von Anspruch 4 abhängig, wobei der erste Abschnitt (116) ein vorderer Abschnitt und der zweite Abschnitt (118) ein hinterer Abschnitt ist, das Verfahren weiter umfassend:
In-Eingriff-Bringen des ersten Abschnitts (116) mit einer Drehsperre (140), um den ersten Abschnitt (116) in Bezug auf den Zylinder (102) drehfest zu fixieren, bevor die Kolbenstange (114) weiter in den Zylinder (102) eingeführt wird, sodass sich der zweite Abschnitt (118) in Bezug auf den ersten Abschnitt (116) dreht.

7. Verfahren nach Anspruch 6, wobei die Drehsperre (140) einen mit einer Passung versehenen Durchlass (142) umfasst, wobei ein Querschnitt mindestens eines Teils der Kolbenstange (114) einer Form des mit einer Passung versehenen Durchlasses (142) entspricht und/oder das Verfahren weiter drehfestes Fixieren des zweiten Abschnitts (118) in Bezug auf den ersten Abschnitt (116) umfasst, nachdem die Kolbenstangenbaugruppe (112) an dem Zylinder (102) angebracht wurde.

8. Verfahren nach Anspruch 7, wobei die Kolbenstangenbaugruppe (112) weiter ein hinteres Element (130) und mindestens einen Schenkel (152a, 152b) umfasst, wobei der mindestens eine Schenkel (152a, 152b) nach Anbringen der Kolbenstangenbaugruppe (112) an dem Zylinder (102) direkt oder indirekt mit dem Zylinder (102) verschiebbar in Eingriff vorliegt,
und wobei drehfestes Fixieren des zweiten Abschnitts (118) in Bezug auf den ersten Abschnitt (116) das In-Eingriff-Bringen des zweiten Abschnitts (118) mit dem hinteren Element (130) umfasst.

9. Verfahren nach Anspruch 8, wenn direkt oder indirekt von Anspruch 6 oder 7 abhängig, wobei die Kolbenstangenbaugruppe (112) weiter ein vorderes Element (148) zum Anbringen an dem Zylinder (102) und mit dem mindestens einen Schenkel (152a, 152b) verschiebbar in Eingriff umfasst, wobei das vordere Element (148) die Drehsperre (140) umfasst.

10. Verfahren nach Anspruch 8 oder 9, wenn von Anspruch 7 abhängig, wobei In-Eingriff-Bringen des zweiten Abschnitts (118) mit dem hinteren Element (130) Bewegen der Kolbenstange (114) nach hinten umfasst.

11. Verfahren nach Anspruch 10, wobei das hintere Element (130) mindestens einen Durchlass (134) zum Aufnehmen mindestens eines sich nach hinten erstreckenden Halteelements (132) des zweiten Abschnitts (118) umfasst und wobei In-Eingriff-Bringen des zweiten Abschnitts (118) mit dem hinteren Element (130) Einführen des mindestens einen Halteelements (132) in den mindestens einen Durchlass (134) umfasst, und optional wobei das Verfahren während weiterer Einführung der Kolbenstange (114) in den Zylinder (102) weiter Verhindern von Einführung des mindestens einen Halteelements (132) in den mindestens einen Durchlass (134) umfasst.

12. Verfahren nach Anspruch 11, wobei Verhindern von Einführung des mindestens einen Halteelements (132) in den mindestens einen Durchlass (134) Verdecken des mindestens einen Durchlasses (134) umfasst und optional wobei Verdecken des mindestens einen Durchlasses (134) Einführen eines Stopfens (180) in den mindestens eine Durchlass (134) umfasst, um zu verhindern, dass das mindestens eine Halteelement (132) des hinteren Abschnitts (130) in den mindestens einen Durchlass (134) eingeführt wird, und nach Anbringen der Kolbenstangenbaugruppe (112) an dem Zylinder (102) optional weiter Entfernen des Stopfens (180) aus dem mindestens einen Durchlass (134) umfasst, um Aufnahme des mindestens einen Halteelements (132) innerhalb des mindestens einen Durchlasses (134) zu erlauben.

## Revendications

1. Procédé d'assemblage d'une seringue préremplie (100) comprenant :
le réglage d'une longueur d'une tige de piston (114) d'un ensemble tige de piston (112) ; et
le montage de l'ensemble tige de piston (112) sur un corps (102) de la seringue (100), le corps (102) présentant une ouverture à une extrémité avant de celui-ci et comprenant un bouchon (104) positionné à l'intérieur et créant un volume entre le bouchon (104) et l'ouverture, le volume étant au moins partiellement rempli d'une substance destinée à être délivrée par la seringue (100),
dans lequel le réglage de la longueur de l'ensemble tige de piston (112) comprend l'insertion au moins partielle de la tige de piston (114) dans le corps (102) de sorte qu'une extrémité avant (124) de la tige de piston (114) entre en contact avec le bouchon (104),
**caractérisé en ce que** le procédé comprend en outre le déplacement de la tige de piston (114) vers l'arrière après réglage de la longueur de la tige de piston (114) et après que l'extrémité avant (124) de la tige de piston (114) entre en contact avec le bouchon (104) pour définir une séparation entre le bouchon (104) et l'extrémité avant (124) de la tige de piston (114), de sorte qu'après assemblage, la longueur de la tige de piston (114) définit une séparation entre le bouchon (104) et l'extrémité avant (124) de la tige de piston (114).

2. Procédé selon la revendication 1, dans lequel le réglage de la longueur de la tige de piston (114) comprend le positionnement d'une première partie (116) de la tige de piston (114) par rapport à une seconde partie (118) de la tige de piston (114).

3. Procédé selon la revendication 2, dans lequel les première et seconde parties (116, 118) de la tige de piston (114) sont couplées et la première partie (116) est mobile axialement par rapport à la seconde partie (118),
et dans lequel le positionnement de la première partie (116) par rapport à la seconde partie (118) comprend le déplacement axial de la première partie (116) par rapport à la seconde partie (118).

4. Procédé selon une quelconque revendication précédente, comprenant une insertion supplémentaire de la tige de piston (114) dans le corps (102), dans lequel l'insertion supplémentaire entraîne une force axiale agissant sur la première partie (116) pour positionner la première partie (116) par rapport à la seconde partie (118), et facultativement dans lequel l'une des première et seconde parties (116, 118) est reçue de manière télescopique dans l'autre des première et seconde parties (116, 118).

5. Procédé selon l'une quelconque des revendications 2 à 4, dans lequel le réglage de la longueur de la tige de piston (114) comprend la rotation d'au moins une de la première partie (116) et de la seconde partie (118) par rapport à l'autre de la première partie (116) et de la seconde partie (118), et facultativement dans lequel le réglage de la longueur de la tige de piston (114) comprend la mise en prise de filetages réciproques (120, 122) de la première partie (116) et de la seconde partie (118) pour convertir la force de rotation en force axiale et vice-versa, et la rotation d'une de la première partie (116) et de la seconde partie (118) par rapport à l'autre de la première partie (116) et de la seconde partie (118).

6. Procédé selon la revendication 5, lorsqu'elle dépend directement ou indirectement de la revendication 4, dans lequel la première partie (116) est une partie avant et la seconde partie (118) est une partie arrière, le procédé comprenant en outre :
la mise en prise de la première partie (116) avec un verrou rotatif (140) pour fixer en rotation la première partie (116) par rapport au corps (102) avant une insertion supplémentaire de la tige de piston (114) dans le corps (102), de sorte que la seconde partie (118) tourne par rapport à la première partie (116).

7. Procédé selon la revendication 6, dans lequel le verrou rotatif (140) comprend une ouverture à clé (142), une section transversale d'au moins une partie de la tige de piston (114) correspondant à une forme de l'ouverture à clé (142), et/ou le procédé comprenant en outre la fixation en rotation de la seconde partie (118) par rapport à la première partie (116) après montage de l'ensemble tige de piston (112) sur le corps (102).

8. Procédé selon la revendication 7, dans lequel l'ensemble tige de piston (112) comprend en outre un élément arrière (130) et au moins une patte (152a, 152b), la au moins une patte (152a, 152b) étant mise en prise de manière coulissante, directement ou indirectement, avec le corps (102) après montage de l'ensemble tige de piston (112) sur le corps (102),
et dans lequel la fixation en rotation de la seconde partie (118) par rapport à la première partie (116) comprend la mise en prise de la seconde partie (118) avec l'élément arrière (130).

9. Procédé selon la revendication 8 lorsqu'elle dépend directement ou indirectement de la revendication 6 ou 7, l'ensemble tige de piston (112) comprenant en outre un élément avant (148) destiné à être monté sur le corps (102) et mis en prise de manière coulissante avec au moins une patte (152a, 152b), l'élément avant (148) comprenant le verrou rotatif (140).

10. Procédé selon la revendication 8 ou 9 lorsqu'elle dépend de la revendication 7, dans lequel la mise en prise de la seconde partie (118) avec l'élément arrière (130) comprend le déplacement de la tige de piston (114) vers l'arrière.

11. Procédé selon la revendication 10, dans lequel l'élément arrière (130) comprend au moins une ouverture (134) destinée à recevoir au moins une broche s'étendant vers l'arrière (132) de la seconde partie (118), et dans lequel la mise en prise de la seconde partie (118) avec l'élément arrière (130) comprend l'insertion de la au moins une broche (132) dans l'au moins une ouverture (134), et facultativement le procédé comprenant en outre le fait d'empêcher l'insertion de la au moins une broche (132) dans l'au moins une ouverture (134) lors de l'insertion supplémentaire de la tige de piston (114) dans le corps (102).

12. Procédé selon la revendication 11, dans lequel le fait d'empêcher l'insertion de la au moins une broche (132) dans l'au moins une ouverture (134) comprend l'obturation de l'au moins une ouverture (134) et facultativement dans lequel l'obturation de l'au moins une ouverture (134) comprend l'insertion d'un bouchon (180) dans l'au moins une ouverture (134) pour empêcher la au moins une broche (132) de la partie arrière (130) d'être insérée dans l'au moins une ouverture (134), et facultativement, comprenant en outre le retrait du bouchon (180) de l'au moins une ouverture (134) après montage de l'ensemble tige de piston (112) sur le corps (102) pour permettre la réception de la au moins une broche (132) au sein de l'au moins une ouverture (134).
